# EUROPEAN PATENT APPLICATION

(11) **EP 3 991 644 A1**
(43) Date of publication of application: **04.05.2022**
(21) Application number: 20020498.0
(22) Date of filing: 29.10.2020
(51) Int. Cl.: A61B 5/021

(54) **METHOD AND APPARATUS FOR THE ELECTRONIC MONITORING OF LIFE-THREATENING CONDITIONS**

(71) Applicant: Cherry Biotech SAS, 35000 Rennes (FR)
(72) Inventor: Homs Corbera, Antoni, 177220 Sant Feliu de Guixols, Girona (ES); Fassini, Dario, 14000 Caen (FR); Cramer, Jeremy, 75014 Paris (FR); Zhu, Tianqi, 59370 Mons-en-Baroeul (FR); Guerinier, Thomas, 35000 Rennes (FR); Velve Casquillas, Guilhem, 75011 Paris (FR); Ion, Marian, Bucharest 051214 (RO); Dinulescu, Silviu, Mun. Pitesti, Jud. Arges 110010 (RO); Moldovan, Carmen Aura, Bucuresti, 011214 (RO); Marin, Iuliana, Bucharest, 052822 (RO); Stanciu, Irina, Bucharest (RO); Goga, Nicolae, Bucuresti (RO); Vasilateanu, Andrei, Bucuresti, 031323 (RO); Scafa Udriste, Alexandru, Bucharest (RO); Ceausu, Iuliania, Bucuresti, 61108 (RO)

(57) **Abstract**

A novel method and electronic device are presented to detect life-threatening conditions through accurate monitoring of blood pressure in subjects. Deflections due to blood pressure oscillations occurring in the tissue of an anatomical part of a subject, which happens to enclose at least one artery or one part of an artery, are electronically sensed by associated deformations of the electronic device once the device is placed in intimate contact with the aforementioned anatomical part. The electronic device contains an electrically conductive fluid, hydrogel or flexible polymer placed in an elastic/flexible non-conductive enclosure and an array of embedded microelectrodes in contact with the electrically conductive part. Deformation of these parts produces an associated change on the electrical conductance or impedance between the electrodes of the array. The physical modification of the distance between the electrodes due to the systolic and diastolic blood pressure fluctuations in the anatomical part produces changes in the electric field occurring between the electrodes while a known DC or AC current or voltage is applied which permit to reach blood pressure data through calibration relationships.

## Description

### TECHNICAL FIELD

This invention relates to detecting and monitoring life-threatening conditions, and more in particular a life-threatening conditions' detecting electronic method and apparatus.

### BACKGROUND

An important life-threatening conditions detection parameter is blood pressure. Blood pressure is generated by the force exerted on the walls of the blood vessels while blood is circulating through them. The highest pressure in the arteries, occurring near the beginning of the cardiac cycle, is known as the systolic pressure. The lowest pressure, happening at the resting phase of the cardiac cycle, is called diastolic pressure. The difference between these two extreme pressures is called the pulse pressure. The mean arterial pressure is calculated by averaging the pressure over a cardiac cycle. These measurements are used as key indicators for several pathological conditions some of them life-threatening.

Different invasive and non-invasive methods to measure blood pressure do exist. Invasive methods rely mainly in penetrating the blood vessels and measuring inside them being generally restricted to hospital settings. Non-invasive methods are less painful and risky, in terms of complications, for the patient. These methods can be oscillometric or auscultatory and, more recently, methods based in optical sensors and flexible optical wave guides have been proposed (see WO2008094340A3). The non-invasive methods are simpler and quicker and more suitable for monitoring and for routine health examinations.

Oscillometric methods use a pressure sensor that is pneumatically connected to an inflatable cuff. The cuff is placed around the upper arm at a vertical height similar to the heart. Initially, the cuff is inflated to reach a pressure substantially above systolic pressure. The cuff is then gradually deflated, and the pressure reduces accordingly. The pressure sensor measures and registers the oscillations of the arterial pressure during the whole process. The amplitude of these oscillations at given cuff pressures is then used to calculate systolic and diastolic pressures. The algorithm used in the calculation is generally set empirically by experimentally setting, in a calibration step, the required coefficients to match the measured by other methods pressures. The oscillometric methods can produce punctual measurements but can be also used for continuous monitoring of the blood pressure.

The auscultatory method is more subjective but also makes use of an inflatable cuff. The method combines a sphygmomanometer, a stethoscope and the examiner or practitioner's ear. The cuff is placed as in the case of the oscillometric methods and connected to an aneroid gauge, to a mercury manometer or to another type of pressure sensing device. The cuff is inflated until the brachial artery is completely occluded. The examiner listens with the stethoscope at a point, distal to the cuff, of the brachial artery while slowly releasing the cuff pressure. The turbulent flow happening when the blood starts moving in the artery creates some audible faint, repetitive, clear tapping sounds known as the Phase I Korotkoff sounds. These first sounds gradually increase in intensity for at least two consecutive beats and the pressure at which these sounds are heart at first is the systolic blood pressure. There are five phases of Korotkoff sounds that can be heard during the cuff pressure releasing process. During the Phase V all sounds completely disappear indicating the diastolic blood pressure. More objective methods to detect and extract the sound data have been possible recently thanks to the application of automated advanced sound processing and computing methods.

Another method presented more recently (see US20110021931A1) is based in a pressure measuring device which includes an optical sensing system with a fixation device adapted to be placed against an anatomical location of a subject containing an artery. The optical sensing system includes an optical waveguide, an optical source device to supply optical energy to the optical waveguide, and an optical detector to detect an amount of optical energy exiting the optical waveguide. The optical sensing system is adapted to sense pulses of the brachial arterial from the compression or flexing of at least a portion of the optical waveguide resulting in reduction of the amount of light exiting the optical waveguide. The output unit is configured to receive a signal indicative of the amount of light exiting the optical waveguide and to generate a measure, based at least in part on the received signal, of a heart rate, of an arterial pulse waveform, a systolic blood pressure, a diastolic blood pressure, a mean arterial blood pressure, a pulse pressure, and an arterial compliance. In order to measure blood pressure can include the application and usage of a varying pressure to an anatomical location of the subject including an artery as in their other noninvasive methods.

Preeclampsia is a live-threatening and pregnancy-specific syndrome estimated to complicate 2 to 8% of all pregnancies [Duley, 2009] and strongly correlated to blood pressure abnormalities. It is characterized by an onset of hypertension and proteinuria occurring after the 20 weeks period of gestation and it affects many organ systems. Preeclampsia can lead to serious, and even fatal, complications for both the carrying mother and the baby if left undetected and untreated. The effects of the syndrome can persist long after the delivery of the baby.

It is still a challenge to classify precisely the diverse hypertensive disorders of pregnancy. This is mainly to nomenclature changes and the geographic variations of the accepted diagnostic criteria. These inconsistencies primary exist in evaluating the degree of hypertension, the presence of proteinuria or the disease severity classification [Steegers et al, 2010]. In the case of severe preeclampsia, blood pressure can reach values over 160 mmHg for systolic blood pressure and over 110 mmHg. For diastolic blood pressure. However, this is not the case in all the severe cases presenting other symptoms and signs in addition to high blood pressure values and proteinuria. Furthermore, subjects with pre-existing hypertension or renal diseases are difficult to diagnose since they show increased blood pressure and urinating protein levels towards the end of the pregnancy period. In any case, currently available methods and classification systems still fail to fully identify all the women that are at high risk of adverse pregnancy outcomes. Preeclampsia can develop with scarce symptoms or not any symptom at all. Some of the common signs of preeclampsia include blood pressure increase but this may develop slowly or alternatively present a sudden onset. Punctual or continuous changes from blood pressure individual basal condition can bring important information on the subjects' condition that is currently not easily available.

Methods targeting to develop clinically relevant and objective definitions guided by evidence and based on predictors are clearly needed. Current state of the art suggests the need of a more continuous monitoring of critical parameters such as blood pressure to efficiently detect those life-threatening conditions which tend to be characterized by the appearance of physio pathological symptoms in a time continuum.

### SUMMARY

Systolic and diastolic blood pressure fluctuations in the patient are translated into deflections of the tissue of the subject's wrist or arm. These deflections translate into a deformation of any elastic or flexible material in close and tied contact with the skin.

The invention includes an electronic sensor which comprises: an electrically conductive fluid, hydrogel or flexible polymer enclosed in an elastic/flexible non-conductive part, an array of embedded microelectrodes in contact with the aforementioned electrically conductive part, a generator of at least one given AC current or voltage and at least one detector of the indirectly generated voltage or current respectively.

The method consists on measuring the deformation of the conductive part, or/and a parameter causing such a deformation, occurring as a consequence of the local deformation of the apparatus and comprises: measuring the impedance' changes at different AC frequencies resulting from the alteration of the electrical path existing between electrodes and created by the conductive part of the apparatus while being deformed, and measuring the blood pressure which generates such deformation after the appropriate calibration and processing of the resulting impedance values. The acquired outputs from the array of electrodes are used to reach blood pressure data through the calibration relationships.

In one embodiment of the invention the sensor and the method can be capable of wirelessly interact securely with a secondary device such as a cell phone or a tablet for the continuous monitoring of blood pressure from a patient to store or process the data to generate important alerts to the user and eventually to the healthcare system connected to it.

### BRIEF DESCRIPTION OF THE DRAWINGS

Other purposes, characteristics and advantages of the invention will emerge more clearly upon reading the following description of a particular embodiment, provided as a simple illustrative and non-restrictive example, in relation to the figures, among which:

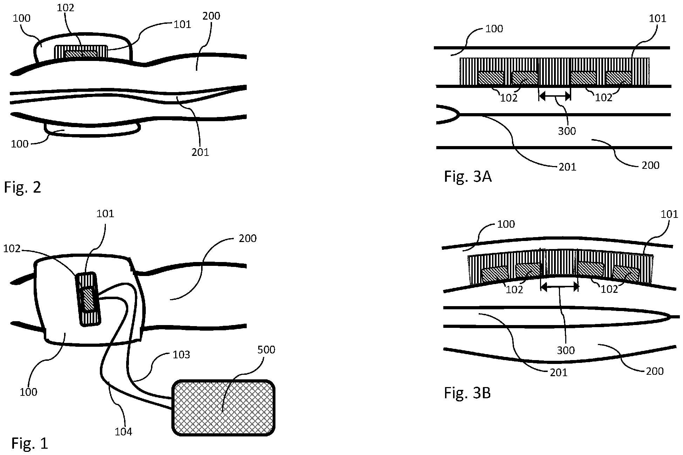

Fig. 1 depicts one implementation of the apparatus for the electronic monitoring of life-threatening conditions.
Fig. 2 depicts the cross-sectional view of the device according to any embodiment of the invention in relation to Fig. 1.
Fig. 3A shows a detailed diagram of a cross-sectional view of a part of a device according to any embodiment of the invention in relation to Fig. 1, and illustrates the device deformation due to its intimate contact with an anatomical part of the monitored subject that contains at least one part of one artery while experimenting a low value of blood pressure.
Fig. 3B shows a detailed diagram of a cross-sectional view of a part of a device according to any embodiment of the invention in relation to Fig. 1, and illustrates the device deformation due to its intimate contact with an anatomical part of the monitored subject that contains at least one part of one artery while experimenting a high value of blood pressure.

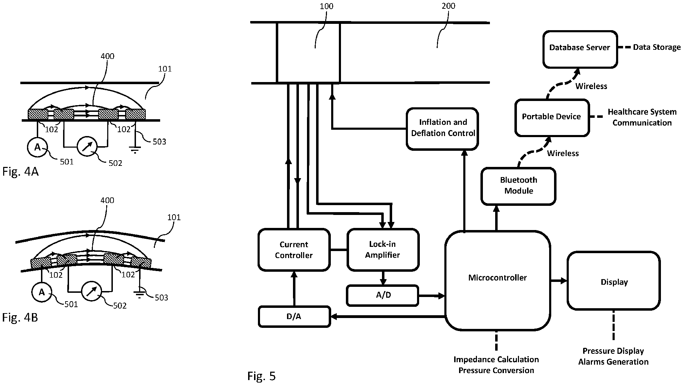

Fig. 4A shows a detailed diagram of a cross-sectional view of a part of an apparatus according to any embodiment of the invention in relation to Fig. 1, when using a 4 points impedance measurement technique, and illustrates the electrical field occurring due to the deformation of the device when is subjected to a low value of blood pressure.
Fig. 4B shows a detailed diagram of a cross-sectional view of a part of an apparatus according to any embodiment of the invention in relation to Fig. 1, when using a 4 points impedance measurement technique, and illustrates the electrical field occurring due to the deformation of the device when is subjected to a high value of blood pressure.
Fig. 5 shows an implementation of one method to detect and monitor one or more physiologic parameters and life-threatening conditions using any embodiment of the invention in relation to Fig. 1.

### DETAILED DESCRIPTION OF THE DRAWINGS (OR EMBODIMENTS)

The general principle of the invention consists in monitoring a subject to detect or predict life-threatening conditions through electrical measurements related to the blood pressure oscillations.

Referring to the drawings in Fig. 1 and Fig. 2, a method and device to monitor electronically life-threatening conditions in accordance to one of the preferred embodiments of the present invention comprises an adapted fixation structure (100) to place a sensor against an anatomical part (200) of the subject which contains at least one part of one artery (201). The sensor, which is of electronic nature, comprises non-electrically conductive elastic body (101) enclosing an electrically conductive fluid, hydrogel, or elastomer. The electrically conductive elastic part is in direct contact with an array of embedded micro electrodes (102), each one with an area smaller than tens of squared millimeters. Electrical stimulations (103) and signals read-out (104) of the electrodes (102) is controlled by a signal generator and measuring unit (500) that may also have signal processing, recording, displaying, or transmitting capabilities.

One method, in accordance with the preferred embodiments of the present invention represented in Fig. 1 and Fig. 2, is further illustrated in Fig. 3A and 3B cross-sectional views. In the sequence, from Fig.3A to Fig. 3B, indirect electrical measurements of the deformation of the elastic sensor (101), or of a physiological parameter causing such a deformation, occurs because of the pressure generated by the anatomical part (200) to the electronic sensor (101) of the apparatus. In Fig. 3A a low value of blood pressure in the artery (201) is illustrated while a high value of blood pressure in the artery (201) is illustrated in Fig. 3B. The anatomical part (200) of the monitored subject generates a pressure on the sensor (101) and its fixation structure (100) and the distance between the microelectrodes (300) is modified as well as the electrical path between them. This modification is monitored as a change in the electrical properties between electrodes which can be processed and calibrated to detect the measurement of a physiological parameter and the detection of life-threatening condition when occurring.

A detailed view of a preferred implementation of the method represented in Fig. 3A and Fig. 3B is illustrated in Fig. 4A and Fig. 4B. In this preferred implementation at least one generator of electrical current (502) is connected to at least 1 microelectrode (102) and at least 1 other microelectrode (102) is grounded (503) generating a closed electrical current path with the electrically conductive elastic part of the sensor (101). The applied DC or AC electrical current generates an electrical field (400) and a measurable voltage between at least 2 of the remaining embedded microelectrodes (102) which are connected to a voltage measuring unit (502) with a high input impedance. A preferred method for this implementation includes the processing of these applied and detected signals to produce a measurement of the changes of the electric field (400) resulting as a consequence of the deformation of the elastic part of the sensor (101) when blood pressure in the artery (201) produces a change in the anatomical part (200). The sequence is illustrated in Fig. 4A and Fig. 4B.

A complete scheme of a preferred embodiment and method of the invention to detect and monitor one or more physiologic parameters and life-threatening conditions is presented in Fig. 5 using any embodiment of the invention in relation to Fig. 1. In this preferred method and embodiment, an adapted fixation structure (100) to place a sensor against an anatomical part (200) can be a cuff comprising and inflatable bladder adapted to apply pressure to a limb (200) when inflated and thereby compressing the artery within the anatomical part (200). In this preferred embodiment of the invention, the processing of the applied and detected electrical signals is used to calculate the impedance' changes resulting from the alteration of the electrical path existing between the micro electrodes and created by the electrically conductive part in contact with the aforementioned micro electrodes as illustrated in Fig. 4A. and Fig. 4B. In this embodiment, an AC current is generated using a current controller by means of a digital to analogic convertor commanded by a microcontroller. A lock-in amplifier, synchronized with the current controller, is used to accurately measure the resulting voltage signal which is received by the microcontroller to accurately calculate the impedance parameters, modulus and phase, using a 4-wires measurement strategy. The microcontroller also controls the inflation and deflation of the adapted fixation structure (100) when is needed and converts the impedance values into blood pressure or other parameters through a previous calibration relationship to detect life-threatening conditions using at least one of those parameters. A display unit is also connected to the microcontroller and used to display important information and alerts to the user of the apparatus. The apparatus is capable of wirelessly interact securely with a secondary portable device, such as a cell phone or a tablet, through Bluetooth Low Energy (BLE) for the continuous monitoring and scouting of potential life-threatening conditions, such as blood pressure, of a subject. The apparatus is also capable of wirelessly interact securely with a third device, such an external server, and to send and receive important alarms and communications to and from the healthcare system by means of an intermediate secondary device such as a cell phone or tablet. The external server can store and process the measurements and data generated by the apparatus to also generate important alerts to the user and eventually to the healthcare institutions. In this preferred embodiment of the invention the monitored life-threatening condition could be preeclampsia.

### INDUSTRIAL APPLICABILITY

The invention is applicable to the healthcare and fitness industrial fields and more in particular as a tool to monitor blood pressure fluctuations in individuals and to early detect life-threatening conditions.

### CITATION LIST

### PATENT LITERATURE

Borgos J. A., Borgos T. A. and Pongratz T. Optical Power Modulation. 2007; WO2008094340A3.
Borgos J. A., Borgos T. A. and Pongratz T. Optical Power Modulation Vital Sign Detection Method and Measurement Device. 2007; US20110021931A1.

### NON-PATENT LITERATURE

Duley L. The Global Impact of Pre-eclampsia and Eclampsia. Semin Perinatol. 2009; 33(3):130-137.
Steegers E. A. P., von Dadelszen P., Duvekot J. J., Pijnenborg R. Pre-eclampsia. The Lancet. 2010; 376(9741): 631-644.

## Claims

1. A novel measurement method and apparatus for monitoring life-threatening related parameters comprising:
an adapted fixation structure to place a sensor against an anatomical part of the subject which contains at least one part of one artery;
an electronic sensor which comprises: an electrically conductive fluid, hydrogel or elastomer, enclosed inside an elastic non-conductive body, and an array of embedded micro electrodes, each one with an area smaller than tens of squared millimeters, in contact with the aforementioned electrically conductive part;
an indirect electrical measurement of the deformation of the conductive part, or of a physiological parameter causing such a deformation, occurring as a consequence of the pressure generated by the anatomical part to the electronic sensor of the apparatus.

2. The method and device according to claim 1, wherein:
at least one generator of at least one given DC or AC current or voltage signal is connected to at least two of the micro electrodes;
at least one detector is connected to the micro electrodes array measuring the voltage or current indirectly resulting during the application of the generated DC or AC currents or voltages to the micro electrodes array;
an output unit processing at least these applied and detected signals to produce a measurement of a life-threatening condition.

3. The method and device according to claim 1, wherein: the conductive elastomer is a Polyurethane-Polypyrrole (PU-PPY) composite, a poly(dimethylsiloxane)-eutectic Gallium-Indium (PDMS-EGaln) structure, a PU-Poly(3,4-ethylenedioxythiophene) (PU-PEDOT) blend, a PU-Poly(3,4-ethylenedioxythiophene) polystyrene sulfonate (PU-PEDOT:PSS) blend, a Graphene-PU composite, or a Silver (Ag)-PU composite.

4. The method and device according to claim 2, wherein: the processing of the applied and detected signals is used to calculate the impedance' changes resulting from the alteration of the electrical path existing between the micro electrodes and created by the electrically conductive part in contact with the aforementioned micro electrodes.

5. The method and device according to claim 4, wherein: impedance is obtained using 4 wires measuring method.

6. The method and device according to claim 4, wherein: impedance is obtained using at least one lock-in amplifier to measure and reduce the noise of at least one measured electrical parameter.

7. The method and device according to claim 2, wherein: the processing of the acquired outputs from the array of micro electrodes is used to measure blood pressure through a previous calibration relationship.

8. The method and device according to claim 1, wherein: the sensor is in close contact with the skin of the subject's arm or wrist in order to monitor at least systolic or diastolic blood pressure fluctuations translating into deflections of the subject's tissue.

9. The method and device according to claim 2, wherein the parameter used to evaluate the life-threatening condition is at least one of the following: arterial pulse waveform, the heart rate, the mean arterial blood pressure, the systolic blood pressure, the diastolic blood pressure, the pulse pressure, or the arterial compliance.

10. The method and device according to claim 2, wherein: the apparatus is capable of wirelessly interact securely with a secondary device, such as a cell phone or a tablet, for the continuous monitoring and scouting of potential life-threatening conditions, such as blood pressure, of a subject.

11. The method and device according to claim 2, wherein: the measurements and data generated by the apparatus are stored or processed to generate important alerts to the user and eventually to the healthcare institutions.

12. The method and device according to claim 1, wherein: the fixation structure is a cuff comprising and inflatable bladder adapted to apply pressure to the limb when inflated and thereby compressing the artery within the limb.

13. The method and device according to claim 2, wherein: the monitored life-threatening condition is preeclampsia, a condition in which a pregnant women or a woman that has just given birth has high blood pressure and signs of damage to other organ systems.
